# EUROPEAN PATENT APPLICATION

(11) **EP 3 257 354 A1**
(43) Date of publication of application: **20.12.2017**
(21) Application number: 17172201.0
(22) Date of filing: 22.05.2017
(51) Int. Cl.: A01G 1/00, A01G 25/16

(54) **PLANT MONITORING SYSTEM AND METHOD**

(30) Priority: 17.06.2016 WO PCT/CN2016/086269
(71) Applicant: Beijing Xiaomi Mobile Software Co., Ltd., Beijing 100085 (CN)
(72) Inventor: FU, Qiang, Beijing, 100085 (CN); ZHOU, Shiquan, Beijing, 100085 (CN); HOU, Enxing, Beijing, 100085 (CN)
(74) Representative: Underwood, Nicolas Patrick

(57) **Abstract**

The present disclosure relates to a plant monitor, a method and an apparatus for generating information and a system for monitoring a plant. The plant monitor (140) comprises a main control chip (141; 241), a wireless communication chip (142; 242) connected with the main control chip (141; 241), and at least one growth environment monitoring sensor (143) connected with the main control chip (141; 241), wherein the at least one growth environment monitoring sensor (143) comprises at least one of an illumination sensor (143a; 243), a temperature sensor (143b; 244), a humidity sensor (143c; 245), and a soil conductivity sensor (143d; 246); the main control chip (141; 241) is configured to acquire growth environment data collected by the at least one growth environment monitoring sensor (143), and to send the growth environment data to a mobile terminal (120) via the wireless communication chip (142; 242), such that the mobile terminal (120) generates plant cultivation reference information according to the growth environment data.

## Description

### TECHNICAL FIELD

Embodiments of the present invention relate to a field of electronic devices, and more particularly, to a plant monitor, a method and an apparatus for generating information, and a system for monitoring a plant.

### BACKGROUND

Ornamental plant cultivation is one of common hobbies in daily life. The ornamental plant may purify air, relax nerves, and promote health excellently.

In a process of cultivating the ornamental plant, a user is required to perform lighting, watering and fertilization on the ornamental plant in accordance with the plant requirements. However, it is difficult for the user to cultivate the ornamental plant to a better state without knowing the plant requirements, because ornamental plants are becoming more and more diversified.

### SUMMARY

In order to solve the problems of the difficulty for the user to cultivate the ornamental plant to the better state without knowing the plant requirements, embodiments of the present invention provide a plant monitor, a method and an apparatus for generating information, and a system for monitoring a plant. The technical solutions may be as follows.

According to a first aspect of the present invention, a plant monitor is provided. The plant monitor includes: a main control chip, a wireless communication chip connected with the main control chip, and at least one growth environment monitoring sensor connected with the main control chip, in which the at least one growth environment monitoring sensor includes at least one of an illumination sensor, a temperature sensor, a humidity sensor, and a soil conductivity sensor;
the main control chip is configured to acquire growth environment data collected by the at least one growth environment monitoring sensor, and to send the growth environment data to a mobile terminal via the wireless communication chip, such that the mobile terminal generates plant cultivation reference information according to the growth environment data.

In a particular embodiment, the plant monitor further includes an insertion arm configured to be inserted into soil, the at least one growth environment monitoring sensor comprising a humidity sensor, and the humidity sensor is arranged on the insertion arm, and configured to collect soil humidity.

In a particular embodiment, the plant monitor further includes an insertion arm configured to insert into soil, the at least one growth environment monitoring sensor comprising a soil conductivity sensor, and the soil conductivity sensor includes two metal electrodes, and the two metal electrodes are arranged on the insertion arm.

In a particular embodiment, the plant monitor further comprises: two insertion arms configured to be inserted into soil, the soil conductivity sensor comprises two metal electrodes, the two insertion arms being arranged in parallel, and each insertion arm (247) is provided with one metal electrode.

In a particular embodiment, the main control chip is further configured to build a binding relationship with the mobile terminal via the wireless communication chip.

According to a second aspect of the present invention, a method for generating information is provided. The method includes:
receiving growth environment data sent by a plant monitor, in which the growth environment data is collected by the plant monitor via a growth environment monitoring sensor, and the growth environment monitoring sensor includes at least one of an illumination sensor, a temperature sensor, a humidity sensor, and a soil conductivity sensor; and
generating plant cultivation reference information according to the growth environment data.

In a particular embodiment, generating plant cultivation reference information according to the growth environment data includes:
acquiring reference environment data corresponding to a predetermined plant;
comparing the growth environment data with the reference environment data; and
generating the plant cultivation reference information according to a comparison result.

In a particular embodiment, the growth environment data includes illumination data, and the reference environment data includes a first threshold and a second threshold, and the first threshold is less than the second threshold; and
generating the plant cultivation reference information according to a comparison result includes:
   when the illumination data or a statistic value of the illumination data is lower than the first threshold, generating first plant cultivation reference information, in which the first plant cultivation reference information is configured to remind enhancing illumination; and
   when the illumination data or the statistic value of the illumination data is higher than the second threshold, generating second plant cultivation reference information, in which the second plant cultivation reference information is configured to remind reducing illumination.
   In a particular embodiment, the growth environment data includes temperature data, and the reference environment data includes a first temperature and a second temperature, and the first temperature is less than the second temperature; and
   generating the plant cultivation reference information according to a comparison result includes:
   when the temperature data or a statistic value of the temperature data is lower than the first temperature, generating third plant cultivation reference information, in which the third plant cultivation reference information is configured to remind enhancing temperature; and
   when the temperature data or the statistic value of the temperature data is higher than the second temperature, generating fourth plant cultivation reference information, in which the fourth plant cultivation reference information is configured to remind reducing temperature.
   In a particular embodiment, the growth environment data includes soil humidity data, and the reference environment data includes a first humidity and a second humidity, and the first humidity is less than the second humidity; and
   generating the plant cultivation reference information according to a comparison result includes:
      when the soil humidity data or a statistic value of the soil humidity data is lower than the first humidity, generating fifth plant cultivation reference information, in which the fifth plant cultivation reference information is configured to remind watering; and
      when the soil humidity data or the statistic value of the soil humidity data is higher than the second humidity, generating sixth plant cultivation reference information, in which the sixth plant cultivation reference information is configured to remind ventilation.

In a particular embodiment, the growth environment data includes a soil PH value, and the soil PH value is acquired by calculating according to the soil conductivity, and the reference environment data includes a first PH value and a second PH value, and the first PH value is less than the second PH value; and
generating the plant cultivation reference information according to a comparison result includes:
   when the soil PH value or a statistic value of the soil PH values is lower than the first PH value, generating seventh plant cultivation reference information, in which the seventh plant cultivation reference information is configured to remind applying alkaline fertilizer; and
   when the soil PH value or the statistic value of the soil PH values is higher than the second PH value, generating eighth plant cultivation reference information, in which the eighth plant cultivation reference information is configured to remind applying acidic fertilizer.
   In a particular embodiment, the growth environment data includes a soil PH value, and the soil PH value is acquired by calculating soil conductivity, and the reference environment data includes a third PH value and a fourth PH value, and the third PH value is less than the fourth PH value; and
   generating the plant cultivation reference information according to a comparison result includes:
      when the soil PH value or a statistic value of the soil PH values is lower than the third PH value, generating ninth plant cultivation reference information, in which the ninth plant cultivation reference information is configured to remind applying first elemental fertilizer, and the first elemental fertilizer has an absorption rate lower than a predetermined condition in acidic soil; and
      when the soil PH value or the statistic value of the soil PH values is higher than the fourth PH value, generating tenth plant cultivation reference information, in which the tenth plant cultivation reference information is configured to remind applying second elemental fertilizer, and the second elemental fertilizer has an absorption rate lower than a predetermined condition in alkaline soil.

In a particular embodiment, the method further includes:
building a binding relationship with the plant monitor; and
recording the predetermined plant corresponding to the plant monitor.

In a particular embodiment, the method further includes:
displaying the plant cultivation reference information and a guidance button on a graphical user interface, in which the guidance button includes at least one of a button for buying a cultivation implement, a button for buying fertilizer, a button for jumping to a cultivation forum, and a button for jumping to a cultivation technique.

According to a third aspect of the present invention, an apparatus for generating information is provided. The apparatus includes:
a receiving module, configured to receive growth environment data sent by a plant monitor, in which the growth environment data is collected by the plant monitor via a growth environment monitoring sensor, and the growth environment monitoring sensor includes at least one of an illumination sensor, a temperature sensor, a humidity sensor, and a soil conductivity sensor; and
a generating module, configured to generate plant cultivation reference information according to the growth environment data.

In a particular embodiment, the generating module includes:
an acquiring sub module, configured to acquire reference environment data corresponding to a predetermined plant;
a comparing sub module, configured to compare the growth environment data with the reference environment data; and
a generating sub module, configured to generate the plant cultivation reference information according to a comparison result.

In a particular embodiment, the growth environment data includes illumination data, and the reference environment data includes a first threshold and a second threshold, and the first threshold is less than the second threshold; and

the generating sub module is configured to generate first plant cultivation reference information when the illumination data or a statistic value of the illumination data is lower than the first threshold, in which the first plant cultivation reference information is configured to remind enhancing illumination; and to generate second plant cultivation reference information when the illumination data or the statistic value of the illumination data is higher than the second threshold, in which the second plant cultivation reference information is configured to remind reducing illumination.

In a particular embodiment, the growth environment data includes temperature data, and the reference environment data includes a first temperature and a second temperature, and the first temperature is less than the second temperature; and
the generating sub module is configured to generate third plant cultivation reference information when the temperature data or a statistic value of the temperature data is lower than the first temperature, in which the third plant cultivation reference information is configured to remind enhancing temperature; and to generate fourth plant cultivation reference information when the temperature data or the statistic value of the temperature data is higher than the second temperature, in which the fourth plant cultivation reference information is configured to remind reducing temperature.

In a particular embodiment, the growth environment data includes soil humidity data, and the reference environment data includes a first humidity and a second humidity, and the first humidity is less than the second humidity; and
the generating sub module is configured to generate fifth plant cultivation reference information when the soil humidity data or a statistic value of the soil humidity data is lower than the first humidity, in which the fifth plant cultivation reference information is configured to remind watering; and to generate sixth plant cultivation reference information when the soil humidity data or the statistic value of the soil humidity data is higher than the second humidity, in which the sixth plant cultivation reference information is configured to remind ventilation.

In a particular embodiment, the growth environment data includes a soil PH value, and the soil PH value is acquired by calculating soil conductivity, and the reference environment data includes a first PH value and a second PH value, and the first PH value is less than the second PH value; and
the generating sub module is configured to generate seventh plant cultivation reference information when the soil PH value or a statistic value of the soil PH values is lower than the first PH value, in which the seventh plant cultivation reference information is configured to remind applying alkaline fertilizer; and to generate eighth plant cultivation reference information when the soil PH value or the statistic value of the soil PH values is higher than the second PH value, in which the eighth plant cultivation reference information is configured to remind applying acidic fertilizer.

In a particular embodiment, the growth environment data includes a soil PH value, and the soil PH value is acquired by calculating soil conductivity, and the reference environment data comprises a third PH value and a fourth PH value, and the third PH value is less than the fourth PH value; and
the generating sub module is configured to generate ninth plant cultivation reference information when the soil PH value or a statistic value of the soil PH values is lower than the third PH value, in which the ninth plant cultivation reference information is configured to remind applying first elemental fertilizer, and the first elemental fertilizer has an absorption rate lower than a predetermined condition in acidic soil; and to generate tenth plant cultivation reference information when the soil PH value or the statistic value of the soil PH values is higher than the fourth PH value, in which the tenth plant cultivation reference information is configured to remind applying second elemental fertilizer, and the second elemental fertilizer has an absorption rate lower than a predetermined condition in alkaline soil.

In a particular embodiment, the apparatus further includes:
a binding module, configured to build a binding relationship with the plant monitor; and
a recording module, configured to record the predetermined plant corresponding to the plant monitor.

In a particular embodiment, the apparatus further includes:
a displaying module, configured to display the plant cultivation reference information and a guidance button on a graphical user interface, in which the guidance button includes at least one of a button for buying a cultivation implement, a button for buying fertilizer, a button for jumping to a cultivation forum, and a button for jumping to a cultivation technique.

According to a fourth aspect of the present invention, an apparatus for generating information is provided. The device includes:
a processor; and
a memory configured to store an instruction executable by the processor;
in which the processor is configured to:
   receive growth environment data sent by a plant monitor, in which the growth environment data is collected by the plant monitor via a growth environment monitoring sensor, and the growth environment monitoring sensor includes at least one of an illumination sensor, a temperature sensor, a humidity sensor, and a soil conductivity sensor; and
   generate plant cultivation reference information according to the growth environment data.

According to a fifth aspect of the present invention, a system for monitoring a plant is provided. The system includes a mobile terminal and a plant monitor,
in which the mobile terminal is connected to the plant monitor via a wireless connection;
the plant monitor is based on the first aspect of the present invention; and
the mobile terminal includes the apparatus for generating information based on the third or fourth aspect of the present invention.

According to a sixth aspect of the present invention, a computer program is provided. When the computer program is executed on a processor of a mobile terminal, a method according to the second aspect of the present invention is performed.

This program can use any programming language and take the form of source code, object code or a code intermediate between source code and object code, such as a partially compiled form, or any other desirable form.

The invention is also directed to a computer-readable information medium containing instructions of a computer program as described above.

The information medium can be any entity or device capable of storing the program. For example, the medium can include storage means such as a ROM, for example a CD ROM or a microelectronic circuit ROM, or magnetic storage means, for example a diskette (floppy disk) or a hard disk.

Alternatively, the information medium can be an integrated circuit in which the program is incorporated, the circuit being adapted to execute the method in question or to be used in its execution.

The technical solutions provided in embodiments of the present invention may have following advantageous effects.

The growth environment data of the plant is collected by the at least one growth environment monitoring sensor, such that the mobile terminal may generate the plant cultivation reference information according to the growth environment data. Therefore, the problems of the difficulty for the user to cultivate the ornamental plant in a better state without knowing the plant requirements may be solved; moreover, it only requires to insert the plant monitor into the plant growth soil, such that the user may acquire the plant cultivation reference information without knowing the plant requirements and without determining appropriateness of the plant growth environment manually.

### BRIEF DESCRIPTION OF THE DRAWINGS

In order to explicitly illustrate embodiments of the present disclosure, a brief introduction for the accompanying drawings corresponding to the embodiments will be listed as follows. Apparently, the drawings described below are only corresponding to some embodiments of the present disclosure, and those skilled in the art may obtain other drawings according to these drawings without creative labor.
Fig. 1 is a schematic diagram illustrating a system for monitoring a plant according to an exemplary embodiment of the present disclosure;
Fig. 2A is a block diagram illustrating a plant monitor according to an exemplary embodiment of the present disclosure;
Fig. 2B is a block diagram illustrating a plant monitor according to another exemplary embodiment of the present disclosure;
Fig. 3 is a flow chart showing a method for generating information according to an exemplary embodiment of the present disclosure;
Fig. 4 is a flow chart showing a method for generating information according to another exemplary embodiment of the present disclosure;
Fig. 5A is a schematic diagram illustrating an interface of a method for generating information according to an exemplary embodiment of the present disclosure;
Fig. 5B is a schematic diagram illustrating an interface of a method for generating information according to an exemplary embodiment of the present disclosure;
Fig. 6 is a flow chart showing a method for generating information according to another exemplary embodiment of the present disclosure;
Fig. 7 is a flow chart showing a method for generating information according to another exemplary embodiment of the present disclosure;
Fig. 8 is a flow chart showing a method for generating information according to another exemplary embodiment of the present disclosure;
Fig. 9 is a flow chart showing a method for generating information according to another exemplary embodiment of the present disclosure;
Fig. 10 is a flow chart showing a method for generating information according to another exemplary embodiment of the present disclosure;
Fig. 11A is a block diagram illustrating an apparatus for generating information according to an exemplary embodiment of the present disclosure;
Fig. 11B is a block diagram illustrating an apparatus for generating information according to another exemplary embodiment of the present disclosure; and
Fig. 12 is a block diagram illustrating a mobile terminal according to an exemplary embodiment of the present disclosure.

### DETAILED DESCRIPTION

In order to make objectives, technical solutions and advantages of the present disclosure clearer, in the following the embodiments of the present disclosure will be described in detail with reference to drawings.

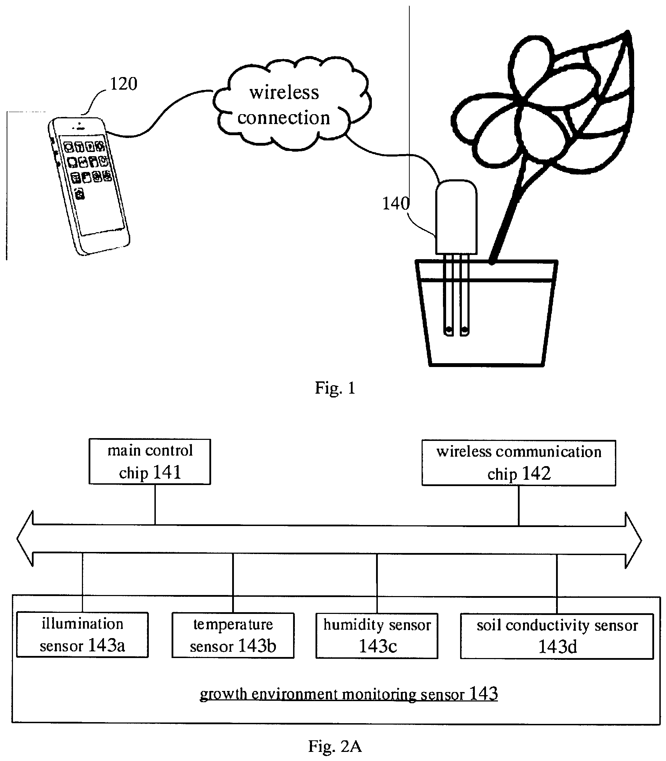

Fig. 1 is a schematic diagram illustrating a system for monitoring a plant according to an exemplary embodiment of the present disclosure. The system includes a mobile terminal 120 and a plant monitor 140.

The mobile terminal 120 may be a mobile phone, a tablet computer, an e-book reader, an MP3 (Moving Picture Experts Group Audio Layer III) player, an MP4 (Moving Picture Experts Group Audio Layer IV) player, a laptop computer, a desktop computer and the like. Alternatively, a plant-cultivation-type application is installed in the mobile terminal 120.

The mobile terminal 120 is connected to the plant monitor 140 via a wireless connection. The wireless connection may be at least one of a Zigbee (Zigzag Flying of Bees) connection, a wireless networking standard Z-Wave connection, a Wi-Fi (Wireless Fidelity) connection, a BLE (Bluetooth Low Energy) connection, and an RF (Radio Frequency) connection.

The plant monitor 140 is an instrument or an apparatus for monitoring growth environment data of the plant and for sending the monitored growth environment data to the mobile terminal 120.

Fig. 2A is a block diagram illustrating a plant monitor 140 according to an exemplary embodiment of the present disclosure. The plant monitor 140 includes a main control chip 141, a wireless communication chip 142 connected with the main control chip 141, and at least one growth environment monitoring sensor 143 connected with the main control chip 141.

The at least one growth environment monitoring sensor 143 includes at least one of an illumination sensor 143a, a temperature sensor 143b, a humidity sensor 143c and a soil conductivity sensor 143d.

The wireless communication chip 142 may be at least one of a Zigbee chip, a Z-Wave chip, a Wi-Fi chip, a BLE chip and an RF chip.

The illumination sensor 143a is configured to collect illumination data of the plant growth environment. The temperature sensor 143b is configured to collect temperature data of the plant growth environment. The humidity sensor 143c is configured to collect air humidity data and/or soil humidity data in the plant growth environment. The soil conductivity sensor 143d is configured to collect electrical conductivity in the plant growth environment. Alternatively, the conductivity is converted into a soil PH value, or the conductivity is converted into a soil fertility index.

The main control chip 141 is configured to acquire growth environment data collected by the growth environment monitoring sensor 143, and to send the growth environment data to the mobile terminal 120 via the wireless communication chip 142. The mobile terminal 120 is configured to generate plant cultivation reference information according to the growth environment data.

Alternatively, the growth environment data includes at least one of the illumination data, the temperature data, the soil humidity data and the PH value.

Alternatively, the main control chip 141 sends the growth environment data to the mobile terminal 120 based on a predetermined transmission strategy. Alternatively, the predetermined transmission strategy includes at least one of sending every predetermined time interval, sending when the data changes, and sending when receiving a data request sent by the mobile terminal.

Alternatively, the main control chip 141 is further configured to build a binding relationship with the mobile terminal 120 via the wireless communication chip 142.

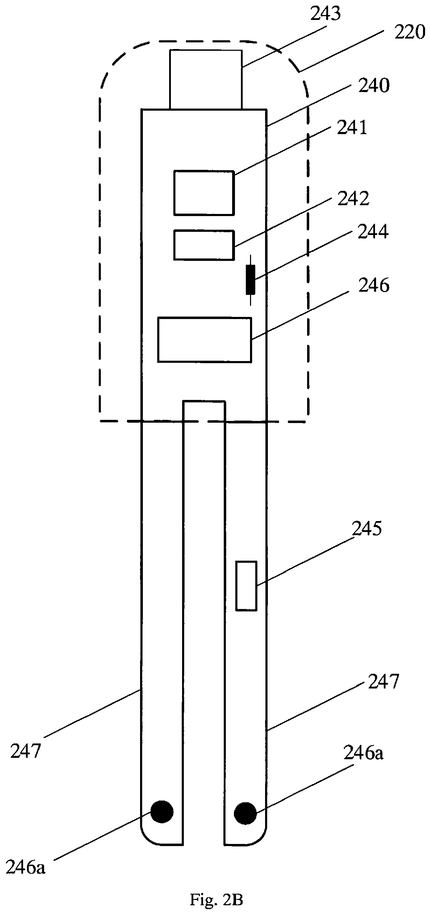

In an illustrative, alternative embodiment, as shown in Fig. 2B, the plant monitor 140 includes a housing 220 and a PCB (Printed circuit board) 240 in the housing 220. The PCB 240 is provided with a main control chip 241, a Bluetooth communication chip 242, an illumination sensor 243, a temperature sensor 244, a humidity sensor 245, and a soil conductivity sensor 246. The PCB 240 also includes conductive traces connecting respective components and peripheral circuitry (not shown).

Alternatively, a lighting component of the illumination sensor 243 corresponds to a transparent region of the housing 220, or the lighting component of the illumination sensor 243 is exposed on the housing 220. Alternatively, the illumination sensor 243 has a measurable light intensity of 100,000 Lux, and the light intensity is measured with an accuracy of 100 Lux.

Alternatively, the temperature sensor 244 is a thermistor arranged on the PCB 240. Alternatively, the temperature sensor 244 has a measurable temperature range from -20 degree Centigrade to 50 degree Centigrade. The temperature is measured with an accuracy of ± 0.5 degree Centigrade.

Alternatively, the plant monitor 140 includes an insertion arm 247 configured to be inserted into soil. The insertion arm 247 is formed by a portion of the PCB 240 extending out of the housing 220. Alternatively, two insertion arms 247 arranged in parallel are provided. Since the PCB 240 has a corrosion-resistant characteristic, the insertion arm 247 still has the corrosion-resistant characteristic after being inserted into the soil for a long period, as compared to a metal insertion arm.

Alternatively, the humidity sensor 245 is arranged on the insertion arm 247. The humidity sensor 245 is configured to collect soil humidity.

Alternatively, the soil conductivity sensor 246 includes two metal electrodes 246a. The two metal electrodes 246a are arranged on the insertion arm 247. If two insertions arms 247 arranged in parallel are provided, each insertion arm 247 is provided with one metal electrode 246a.

As will be appreciated by those skilled in the art, the plant monitor 140 also includes more or fewer components than the illustrations above. For example, the plant monitor 140 may also include a power source for supplying power to various components of the plant monitor 140. The power supply may be a coin cell battery or a rechargeable battery.

In conclusion, with the plant monitor provided in the present embodiment, the growth environment data of the plant is collected by the at least one growth environment monitoring sensor, such that the mobile terminal may generate the plant cultivation reference information according to the growth environment data. Therefore, the problems that it is difficult for the user to cultivate the ornamental plant to the better state without knowing the plant requirements may be solved; moreover, it only requires to insert the plant monitor into the plant growth soil, such that the user may acquire the plant cultivation reference information without knowing the plant requirements and without determining appropriateness of the plant growth environment manually.

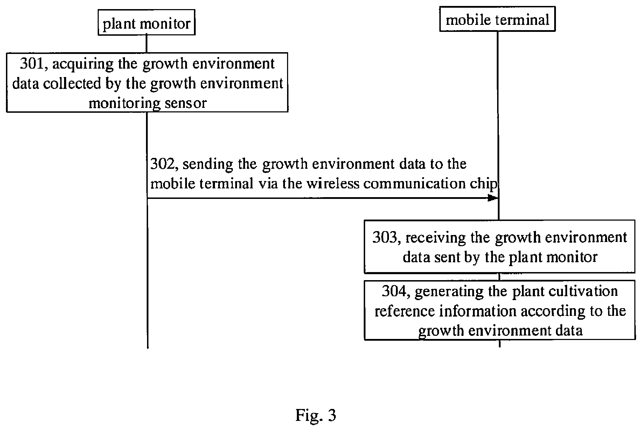

Fig. 3 is a flow chart showing a method for generating information according to an exemplary embodiment of the present disclosure. The present embodiment is exemplified by applying the method for generating information to the system for monitoring a plant shown in Fig. 1. The method includes followings.

In step 301, the plant monitor acquires the growth environment data collected by the growth environment monitoring sensor.

Alternatively, the growth environment data includes at least one of the illumination data, the temperature data, the soil humidity data and the PH value.

In step 302, the plant monitor sends the growth environment data to the mobile terminal via the wireless communication chip.

Alternatively, the plant monitor sends the growth environment data to the mobile terminal via the wireless communication chip every predetermined time interval. Alternatively, when monitoring that the growth environment data changes, the plant monitor sends the growth environment data to the mobile terminal via the wireless communication chip. Alternatively, when receiving a data request sent by the mobile terminal, the plant monitor sends the growth environment data to the mobile terminal via the wireless communication chip.

In step 303, the mobile terminal receives the growth environment data sent by the plant monitor.

In step 304, the mobile terminal generates the plant cultivation reference information according to the growth environment data.

Alternatively, the plant cultivation reference information includes: information configured to remind enhancing illumination, information configured to remind that illumination is appropriate, information configured to remind reducing illumination, information configured to remind enhancing temperature, information configured to remind that the temperature is appropriate, information configured to remind reducing temperature, information configured to remind watering, information configured to remind that the quantity of water is proper, information configured to remind ventilation, information configured to remind applying alkaline fertilizer, information configured to remind that the fertilizer is proper, information configured to remind applying acidic fertilizer, information configured to remind applying first elemental fertilizer, and information configured to remind applying second elemental fertilizer.

The first elemental fertilizer has an absorption rate lower than a predetermined condition in acidic soil. The second elemental fertilizer has an absorption rate lower than a predetermined condition in alkaline soil.

In conclusion, with the method for generating information provided in the present embodiment, the growth environment data of the plant is collected by the at least one growth environment monitoring sensor in the plant monitor, and the growth environment data is sent to the mobile terminal via the wire communication chip, such that the mobile terminal generates the plant cultivation reference information according to the growth environment data. Therefore, the problems that it is difficult for the user to cultivate the ornamental plant in the better state without knowing the plant requirements may be solved; moreover, it only requires to insert the plant monitor into the soil, such that the user may acquire the plant cultivation reference information without knowing the plant requirements and without determining appropriateness of the plant growth environment manually.

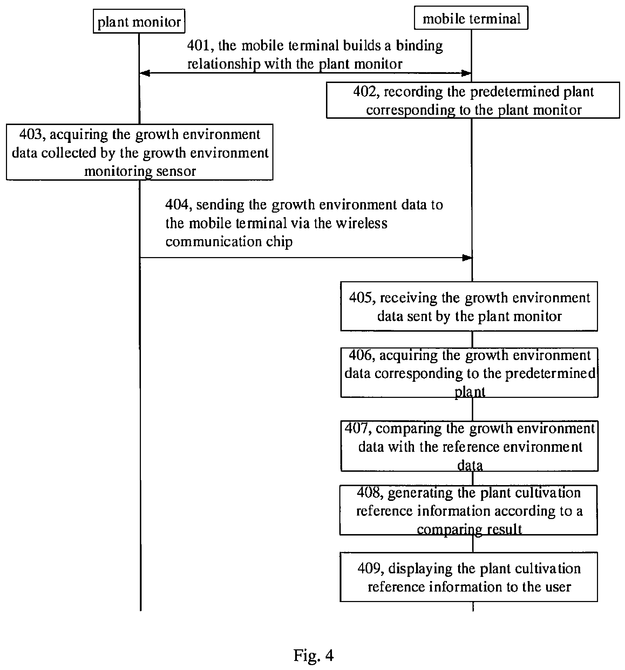

Fig. 4 is a flow chart showing a method for generating information according to an exemplary embodiment of the present disclosure. The present embodiment is exemplified by applying the method for generating information to the system for monitoring a plant shown in Fig. 1. The method includes followings.

In step 401, the mobile terminal builds a binding relationship with the plant monitor.

Alternatively, the mobile terminal builds the binding relationship with the plant monitor by followings.

First, the plant monitor sends a Bluetooth broadcasting message after the initial starting up.

Second, the mobile terminal receives the Bluetooth broadcasting message, and acquires an identifier of the plant monitor according to the Bluetooth broadcasting message.

Third, the mobile terminal binds the identifier of the plant monitor with itself.

After binding, the mobile terminal only receives the growth environment data reported by the bound plant monitor.

The embodiment of the present disclosure does not limit specific forms of the binding process.

In step 402, the mobile terminal records the predetermined plant corresponding to the plant monitor.

Alternatively, in the binding process, the mobile terminal displays an information input interface for requesting the user to input the predetermined plant corresponding to the plant monitor. The predetermined plant is a plant in growth environment monitored by the plant monitor.

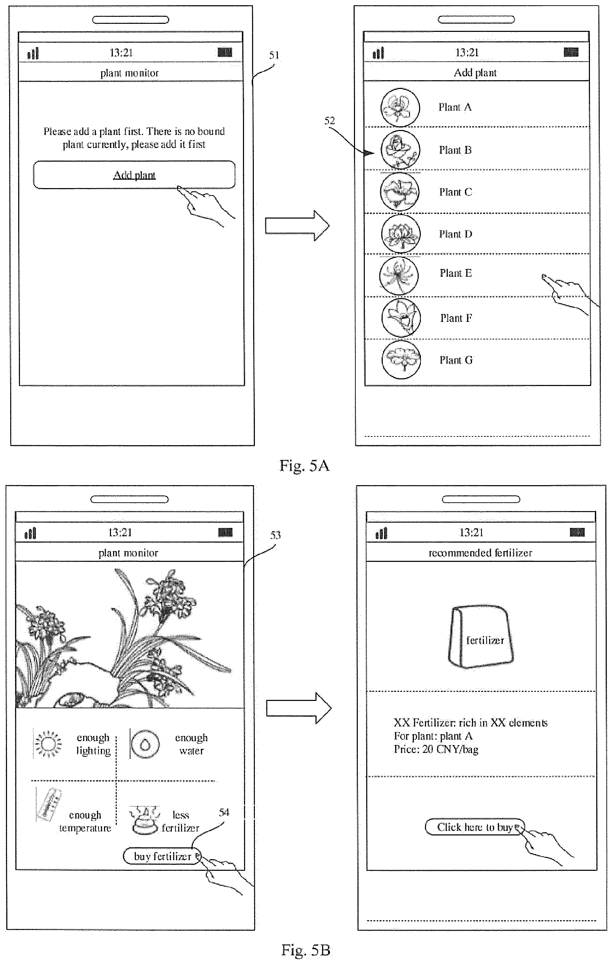

As shown in Fig. 5A, after binding with the plant monitor, the mobile terminal 51 displays an information input interface including the plant selection item 52 for the user to input the predetermined plant corresponding to the plant monitor.

In step 403, the plant monitor acquires the growth environment data collected by the growth environment monitoring sensor.

Alternatively, the growth environment data includes at least one of the illumination data, the temperature data, the soil humidity data and the PH value.

In step 404, the plant monitor sends the growth environment data to the mobile terminal via the wireless communication chip.

Alternatively, the plant monitor sends the growth environment data to the mobile terminal via the wireless communication chip every predetermined time interval.

Alternatively, when monitoring that the growth environment data changes, the plant monitor sends the growth environment data to the mobile terminal via the wireless communication chip.

Alternatively, when receiving a data request sent by the mobile terminal, the plant monitor sends the growth environment data to the mobile terminal via the wireless communication chip.

Alternatively, the predetermined time interval is 15 minutes, 30 minutes, 1 hour, 2 hours, 4 hours, etc.

In step 405, the mobile terminal receives the growth environment data sent by the plant monitor.

Alternatively, the mobile terminal receives the growth environment data sent by the plant monitor via the wire communication chip.

In step 406, the mobile terminal acquires the growth environment data corresponding to the predetermined plant.

Alternatively, the predetermined plant is a plant previously set by the user. The predetermined plant is a plant cultivated in the plant growth environment monitored by the plant monitor.

Alternatively, different plants correspond to different reference environmental data. In an alternative embodiment, each plant has reference environmental data varied with growth stages.

Alternatively, the reference environmental data corresponding to the predetermined plant includes at least one of the reference illumination data, the reference temperature data, the reference air humidity data, the reference soil humidity data, and the reference soil pH value.

In step 407, the mobile terminal compares the growth environment data with the reference environment data.

The mobile terminal compares the collected growth environment data, the statistic value of the growth environment data, or the conversion value of the growth environment data with the reference environment data.

Alternatively, when the growth environment data includes the illumination data, the illumination data or the statistic value of the illumination data includes at least one of: the maximum light intensity, the average light intensity, the minimum light intensity, the daily lighting duration, the daily average light intensity, the daily average lighting duration, the week average light intensity, the week average lighting duration, the month average light intensity, and the month average lighting duration.

Alternatively, when the growth environment data includes the temperature data, the temperature data or the statistic value of the temperature data includes at least one of: the maximum temperature, the average temperature, the minimum temperature, the daytime average temperature, the nighttime average temperature, the week average temperature, and the month average temperature.

Alternatively, when the growth environment data includes the soil humidity data, the soil humidity data or the statistic value of the soil humidity data includes at least one of: the maximum soil humidity, the average soil humidity, the minimum soil humidity, the daytime average soil humidity, the nighttime average soil humidity, the week average soil humidity and the month average soil humidity.

Alternatively, when the growth environment data includes the soil conductivity, the soil conductivity or the statistic value of the soil conductivity or the conversion value of the soil conductivity includes at least one of: the soil salinity, the soil PH value, the soil fertility, the daily average soil salinity, the daily average soil PH value, the daily average soil fertility, the week average soil salinity, the week average soil PH value, the week average soil fertility, the month average soil salinity, the month average soil PH value and the month average soil fertility.

There are conversion relationships between the soil conductivity and the soil salinity, between the soil conductivity and the soil PH value, and between the soil conductivity and the soil fertility. These conversion relationships may be denoted by functions, curves, data tables and other forms.

The mobile terminal may compare the various data in the growth environment data with the corresponding data item in the reference environment data.

In step 408, the mobile terminal generates the plant cultivation reference information according to a comparison result.

In step 409, the mobile terminal displays the plant cultivation reference information to the user.

The mobile terminal displays the plant cultivation reference information in the plant-cultivation-type application. Illustratively, the plant cultivation reference information includes: adequate light, adequate water, adequate temperature, less fertility and other reference information.

Alternatively, in combination with Fig. 5B, the mobile terminal simultaneously displays the plant cultivation reference information and a guidance button 54 on a graphical user interface 53. The guidance button 54 includes at least one of a button for buying a cultivation implement, a button for buying fertilizer, a button for jumping to a cultivation forum, and a button for jumping to a cultivation technique.

In conclusion, with the method for generating information provided in the present embodiment, the mobile terminal may compare the growth environment data of the plant with the reference environment data corresponding to the plant and generate the plant cultivation reference information based on the comparison result. Therefore, for each plant, the corresponding plant cultivation reference information may be generated with high reference value.

In an alternative embodiment, the growth environment data includes illumination data, and the reference environment data includes a first threshold and a second threshold, in which the first threshold is less than the second threshold.

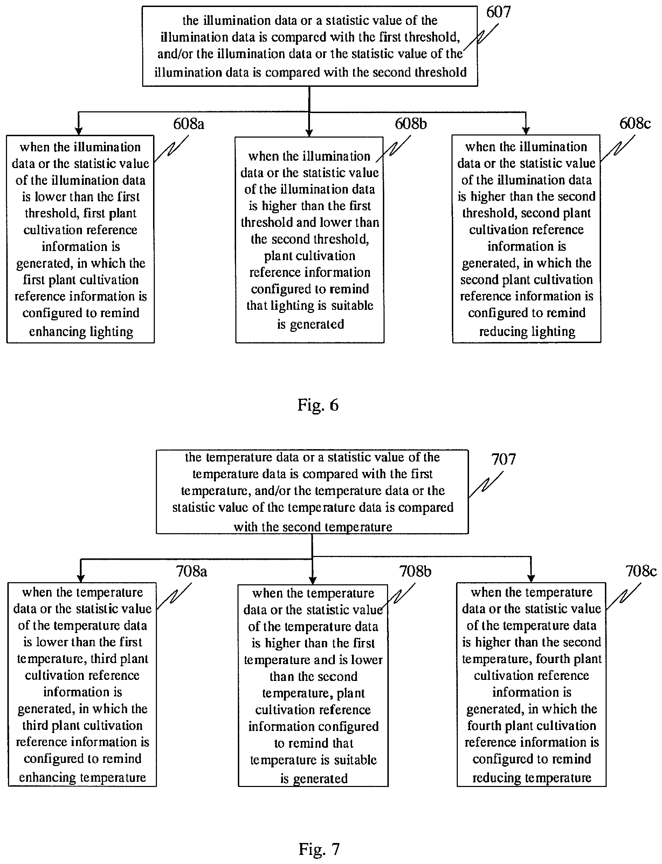

Following steps may be implemented instead of steps 407 and 408, as shown in Fig. 6.

In step 607, the illumination data or a statistic value of the illumination data is compared with the first threshold, and/or the illumination data or the statistic value of the illumination data is compared with the second threshold.

For example, the illumination data includes the daily average lighting duration, the first threshold is the minimum daily average lighting duration, and the second threshold is the maximum daily average lighting duration.

In step 608a, when the illumination data or the statistic value of the illumination data is lower than the first threshold, first plant cultivation reference information is generated, in which the first plant cultivation reference information is configured to remind enhancing lighting.

In step 608b, when the illumination data or the statistic value of the illumination data is higher than the first threshold and lower than the second threshold, plant cultivation reference information configured to remind that the lighting is suitable is generated.

In step 608c, when the illumination data or the statistic value of the illumination data is higher than the second threshold, second plant cultivation reference information is generated, in which the second plant cultivation reference information is configured to remind reducing lighting.

In conclusion, with the method for generating information provided in the present embodiment, the illumination data is compared with the first threshold and the second threshold in the reference environment data, such that the light-related plant cultivation reference information is generated, thus avoiding stunting the plant because of lacking of lighting or excessive lighting.

In an alternative embodiment, the growth environment data includes temperature data, and the reference environment data includes a first temperature and a second temperature, in which the first temperature is less than the second temperature.

Following steps may be implemented instead of steps 407 and 408, as shown in Fig. 7.

In step 707, the temperature data or a statistic value of the temperature data is compared with the first temperature, and/or the temperature data or the statistic value of the temperature data is compared with the second temperature.

For example, the temperature data includes the daily average temperature, the first temperature is the minimum daily average temperature, and the second temperature is the maximum daily average temperature.

In step 708a, when the temperature data or the statistic value of the temperature data is lower than the first temperature, third plant cultivation reference information is generated, in which the third plant cultivation reference information is configured to remind enhancing temperature.

In step 708b, when the temperature data or the statistic value of the temperature data is higher than the first temperature and is lower than the second temperature, plant cultivation reference information configured to remind that the temperature is appropriate is generated.

In step 708c, when the temperature data or the statistic value of the temperature data is higher than the second temperature, fourth plant cultivation reference information is generated, in which the fourth plant cultivation reference information is configured to remind reducing temperature.

In conclusion, with the method for generating information provided in the present embodiment, the temperature data is compared with the first temperature and the second temperature in the reference environment data, such that the temperature-related plant cultivation reference information is generated, thereby avoiding stunting the plant because of too low temperature or too high temperature.

In an alternative embodiment, the growth environment data includes soil humidity data, and the reference environment data includes a first humidity and a second humidity, in which the first humidity is less than the second humidity.

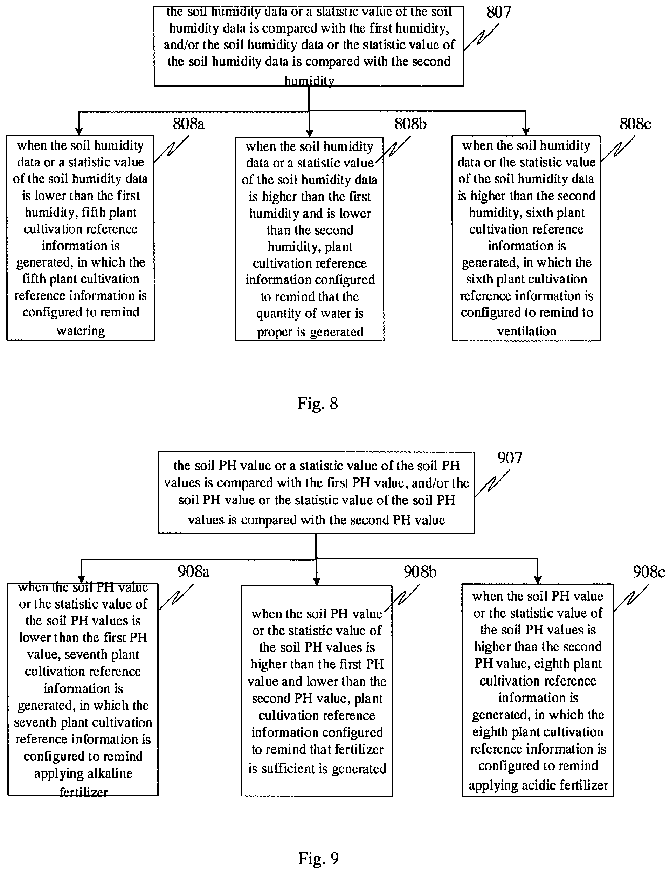

Following steps may be implemented instead of steps 407 and 408, as shown in Fig. 8.

In step 807, the soil humidity data or a statistic value of the soil humidity data is compared with the first humidity, and/or the soil humidity data or the statistic value of the soil humidity data is compared with the second humidity.

For example, the soil humidity data includes the daily average humidity, the first humidity is the minimum daily average humidity, and the second humidity is the maximum daily average humidity.

In step 808a, when the soil humidity data or a statistic value of the soil humidity data is lower than the first humidity, fifth plant cultivation reference information is generated, in which the fifth plant cultivation reference information is configured to remind watering.

In step 808b, when the soil humidity data or a statistic value of the soil humidity data is higher than the first humidity and is lower than the second humidity, plant cultivation reference information configured to remind that the quantity of water is suitable is generated.

In step 808c, when the soil humidity data or the statistic value of the soil humidity data is higher than the second humidity, sixth plant cultivation reference information is generated, in which the sixth plant cultivation reference information is configured to remind ventilation.

In conclusion, with the method for generating information provided in the present embodiment, the soil humidity data is compared with the first humidity and the second humidity in the reference environment data, such that the moisture-related plant cultivation reference information is generated, thereby avoiding stunting the plant because of too litter water or too much water.

In an alternative embodiment, the growth environment data includes a soil PH value, and the reference environment data includes a first PH value and a second PH value, in which the first PH value is less than the second PH value.

Following steps may be implemented instead of steps 407 and 408, as shown in Fig. 9.

In step 907, the soil PH value or a statistic value of the soil PH values is compared with the first PH value, and/or the soil PH value or the statistic value of the soil PH values is compared with the second PH value.

The soil PH value is converted from the soil conductivity. For example, the soil PH value includes the daily average PH value, the first PH value is an acidic PH threshold related to the plant, and the second PH value is an alkali PH threshold related to the plant.

In step 908a, when the soil PH value or the statistic value of the soil PH values is lower than the first PH value, seventh plant cultivation reference information is generated, in which the seventh plant cultivation reference information is configured to remind to apply alkaline fertilizer.

In step 908b, when the soil PH value or the statistic value of the soil PH values is higher than the first PH value and lower than the second PH value, plant cultivation reference information configured to remind that fertilizer is suitable is generated.

In step 908c, when the soil PH value or the statistic value of the soil PH values is higher than the second PH value, eighth plant cultivation reference information is generated, in which the eighth plant cultivation reference information is configured to remind applying acidic fertilizer.

In conclusion, with the method for generating information provided in the present embodiment, the soil PH value is compared with the first PH value and the second PH value in the reference environment data, such that the plant cultivation reference information related to the acidity and alkaline fertilizer can be generated, thereby avoiding stunting the plant because some kind of fertilizer is too little or too much.

In an alternative embodiment, the growth environment data includes a soil PH value, and the reference environment data includes a third PH value and a fourth PH value, in which the third PH value is less than the fourth PH value.

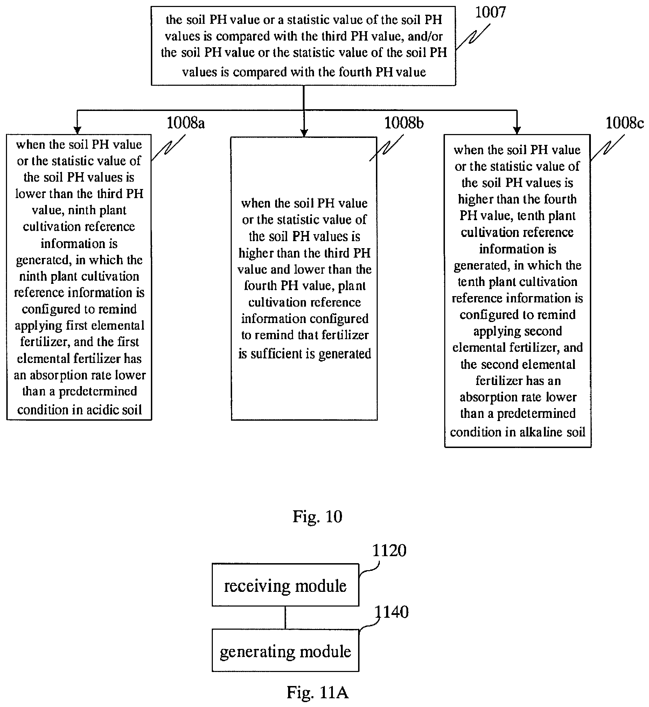

Following steps may be implemented instead of steps 407 and 408, as shown in Fig. 10.

In step 1007, the soil PH value or a statistic value of the soil PH values is compared with the third PH value, and/or the soil PH value or the statistic value of the soil PH values is compared with the fourth PH value.

The soil PH value is converted from the soil conductivity. For example, the soil PH value includes the daily average PH value, the third PH value is an acidic PH threshold related to the plant, and the fourth PH value is an alkali PH threshold related to the plant.

In step 1008a, when the soil PH value or the statistic value of the soil PH values is lower than the third PH value, ninth plant cultivation reference information is generated, in which the ninth plant cultivation reference information is configured to remind applying first elemental fertilizer, and the first elemental fertilizer has an absorption rate lower than a predetermined condition in acidic soil.

For example, the plant in the acidic soil cannot absorb N, P, K and other nutrients well for normal growth, and therefore the user is recommended to apply the fertilizer rich with N, P, K and other nutrients.

In step 1008b, when the soil PH value or the statistic value of the soil PH values is higher than the third PH value and lower than the fourth PH value, plant cultivation reference information configured to remind that fertilizer is suitable is generated.

In step 1008c, when the soil PH value or the statistic value of the soil PH values is higher than the fourth PH value, tenth plant cultivation reference information is generated, in which the tenth plant cultivation reference information is configured to remind applying second elemental fertilizer, and the second elemental fertilizer has an absorption rate lower than a predetermined condition in alkaline soil.

In conclusion, with the method for generating information provided in the present embodiment, the soil PH value is compared with the third PH value and the fourth PH value in the reference environment data, such that the plant cultivation reference information related to nutritional element fertilizer can be generated, thereby avoiding stunting the plant because some kind of fertilizer is too little or too much.

It is to be noted that the above-described alternative embodiments may be implemented in combination with each other, and the embodiments of the present disclosure are not limited thereto.

It is to be noted that the steps performed by the plant monitor in each of the above-described method embodiments may be realized individually as methods for pushing information on the side of the plant monitor; the steps performed by the mobile terminal in each of the above-described method embodiments may be implemented individually as methods for displaying information on the side of the mobile terminal.

The following are apparatus embodiments of the present disclosure, which may be used to implement embodiments of the disclosed method. For details not disclosed in the apparatus embodiments of the present disclosure, reference is made to the method embodiments of the present disclosure.

With respect to the apparatus in the following embodiment, the specific manners in which each module performs the operation has been described in detail in the embodiments relating to the method, and will not be described in detail herein.

Fig. 11A is a block diagram illustrating an apparatus for generating information according to an exemplary embodiment of the present disclosure. The apparatus for generating information may be implemented as a whole or a part of a mobile terminal by a dedicated hardware circuit or a combination of hardware and software. The apparatus includes: a receiving module 1120 and a generating module 1140.

The receiving module 1120 is configured to receive growth environment data sent by a plant monitor, in which the growth environment data is collected by the plant monitor via a growth environment monitoring sensor, and the growth environment monitoring sensor includes at least one of an illumination sensor, a temperature sensor, a humidity sensor, and a soil conductivity sensor.

The generating module 1140 is configured to generate plant cultivation reference information according to the growth environment data.

In conclusion, with the apparatus for generating information provided in the present embodiment, the growth environment data of the plant is collected by the at least one growth environment monitoring sensor in the plant monitor, and the growth environment data is sent to the mobile terminal via the wire communication chip, such that the mobile terminal generates the plant cultivation reference information according to the growth environment data. Therefore, the problems that it is difficult for the user to cultivate the ornamental plant to the better state without knowing the plant requirements may be solved; moreover, it only requires the plant monitor to be inserted into soil for realizing the effect that the user may acquire the plant cultivation reference information without knowing the plant requirements and without determining appropriateness of the plant growth environment manually.

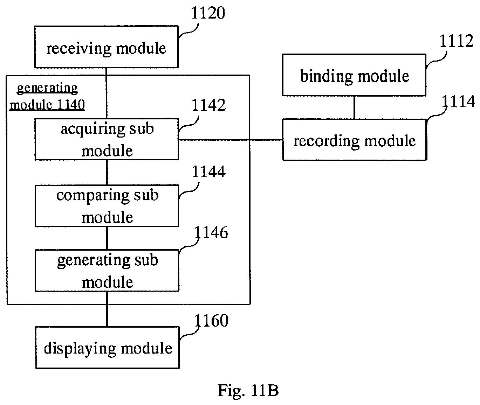

In an alternative embodiment, referring to Fig. 11B, the generating module 1140 includes an acquiring sub module 1142, a comparing sub module 1144 and a generating sub module 1146.

The acquiring sub module 1142 is configured to acquire reference environment data corresponding to a predetermined plant.

The comparing sub module 1144 is configured to compare the growth environment data with the reference environment data.

The generating sub module 1146 is configured to generate the plant cultivation reference information according to a comparison result.

In an alternative embodiment, the growth environment data includes illumination data, and the reference environment data includes a first threshold and a second threshold, in which the first threshold is less than the second threshold.

The generating sub module 1146 is configured to generate first plant cultivation reference information when the illumination data or a statistic value of the illumination data is lower than the first threshold, in which the first plant cultivation reference information is configured to remind enhancing lighting; and to generate second plant cultivation reference information when the illumination data or the statistic value of the illumination data is higher than the second threshold, in which the second plant cultivation reference information is configured to remind reducing lighting.

In an alternative embodiment, the growth environment data includes temperature data, and the reference environment data includes a first temperature and a second temperature, in which the first temperature is less than the second temperature.

The generating sub module 1146 is configured to generate third plant cultivation reference information when the temperature data or a statistic value of the temperature data is lower than the first temperature, in which the third plant cultivation reference information is configured to remind enhancing temperature; and to generate fourth plant cultivation reference information when the temperature data or the statistic value of the temperature data is higher than the second temperature, in which the fourth plant cultivation reference information is configured to remind reducing temperature.

In an alternative embodiment, the growth environment data includes soil humidity data, and the reference environment data includes a first humidity and a second humidity, in which the first humidity is less than the second humidity.

The generating sub module 1146 is configured to generate fifth plant cultivation reference information when the soil humidity data or a statistic value of the soil humidity data is lower than the first humidity, in which the fifth plant cultivation reference information is configured to remind watering; and to generate sixth plant cultivation reference information when the soil humidity data or the statistic value of the soil humidity data is higher than the second humidity, in which the sixth plant cultivation reference information is configured to remind ventilation.

In an alternative embodiment, the growth environment data includes a soil PH value, and the soil PH value is acquired by calculating soil conductivity, and the reference environment data includes a first PH value and a second PH value, in which the first PH value is less than the second PH value.

The generating sub module 1146 is configured to generate seventh plant cultivation reference information when the soil PH value or a statistic value of the soil PH values is lower than the first PH value, in which the seventh plant cultivation reference information is configured to remind applying alkaline fertilizer; and to generate eighth plant cultivation reference information when the soil PH value or the statistic value of the soil PH values is higher than the second PH value, in which the eighth plant cultivation reference information is configured to remind applying acidic fertilizer.

In an alternative embodiment, the growth environment data includes a soil PH value, and the soil PH value is acquired by calculating according to the soil conductivity, and the reference environment data includes a third PH value and a fourth PH value, in which the third PH value is less than the fourth PH value.

The generating sub module 1146 is configured to generate ninth plant cultivation reference information when the soil PH value or a statistic value of the soil PH values is lower than the third PH value, in which the ninth plant cultivation reference information is configured to remind applying first elemental fertilizer, and the first elemental fertilizer has an absorption rate lower than a predetermined condition in acidic soil; and to generate tenth plant cultivation reference information when the soil PH value or the statistic value of the soil PH values is higher than the fourth PH value, in which the tenth plant cultivation reference information is configured to remind applying second elemental fertilizer, and the second elemental fertilizer has an absorption rate lower than a predetermined condition in alkaline soil.

In an alternative embodiment, referring to Fig. 11B, the apparatus further includes a binding module 1112, and a recording module 1114.

The binding module 1112 is configured to build a binding relationship with the plant monitor.

The recording module 1114 is configured to record the predetermined plant corresponding to the plant monitor.

In an alternative embodiment, referring to Fig. 11B, the apparatus further includes a displaying module 1160.

The displaying module 1160 is configured to display the plant cultivation reference information and a guidance button on a graphical user interface, in which the guidance button includes at least one of a button for buying a cultivation implement, a button for buying fertilizer, a button for jumping to a cultivation forum, and a button for jumping to a cultivation technique.

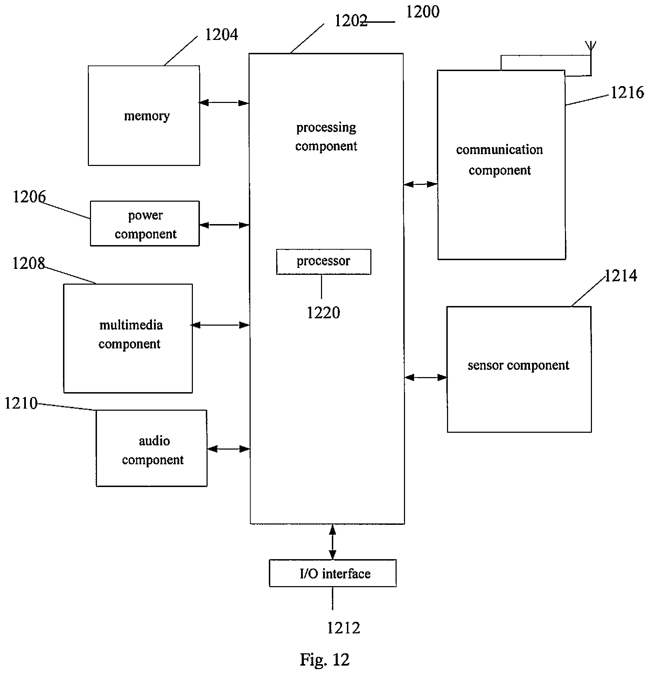

Fig. 12 is a block diagram illustrating a mobile terminal according to an exemplary embodiment of the present disclosure. For example, the mobile terminal 1200 may be a mobile phone, a computer, a digital broadcasting terminal, a messaging device, a game console, a tablet device, a medical device, fitness equipment, a Personal Digital Assistant PDA, and the like

Referring to Fig. 12, the mobile terminal 1200 may include the following one or more components: a processing component 1202, a memory 1204, a power component 1206, a multimedia component 1208, an audio component 1210, an Input/Output (I/O) interface 1212, a sensor component 1214, and a communication component 1216.

The processing component 1202 typically controls overall operations of the mobile terminal 1200, such as the operations associated with display, telephone calls, data communications, camera operations, and recording operations. The processing component 1202 may include one or more processors 1220 to execute instructions to perform all or part of the steps in the above described methods. Moreover, the processing component 1202 may include one or more modules which facilitate the interaction between the processing component 1202 and other components. For instance, the processing component 1202 may include a multimedia module to facilitate the interaction between the multimedia component 1208 and the processing component 1202.

The memory 1204 is configured to store various types of data to support the operation of the mobile terminal 1200. Examples of such data include instructions for any applications or methods operated on the mobile terminal 1200, contact data, phonebook data, messages, pictures, video, etc. The memory 1204 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 1206 provides power to various components of the mobile terminal 1200. The power component 1206 may include a power management system, one or more power sources, and any other components associated with the generation, management, and distribution of power in the mobile terminal 1200.

The multimedia component 1208 includes a screen providing an output interface between the mobile terminal 1200 and the user. In some embodiments, the screen may include a liquid crystal display (LCD) and a press panel (TP). If the screen includes the press panel, the screen may be implemented as a press screen to receive input signals from the user. The press panel includes one or more press sensors to sense presses, swipes, and other gestures on the press panel. The press sensors may not only sense a boundary of a press or swipe action, but also sense a duration time and a pressure associated with the press or swipe action. In some embodiments, the multimedia component 1208 includes a front camera and/or a rear camera. The front camera and/or the rear camera may receive external multimedia data while the mobile terminal 1200 is in an operation mode, such as a photographing mode or a video mode. Each of the front camera and the rear camera may be a fixed optical lens system or have focus and optical zoom capability.

The audio component 1210 is configured to output and/or input audio signals. For example, the audio component 1210 includes a microphone (MIC) configured to receive an external audio signal when the mobile terminal 1200 is in an operation mode, such as a call mode, a recording mode, and a voice recognition mode. The received audio signal may be further stored in the memory 1204 or transmitted via the communication component 1216. In some embodiments, the audio component 1210 further includes a speaker to output audio signals.

The I/O interface 1212 provides an interface for the processing component 1202 and peripheral interface modules, such as a keyboard, a click wheel, buttons, and the like. The buttons may include, but are not limited to, a home button, a volume button, a starting button, and a locking button.

The sensor component 1214 includes one or more sensors to provide status assessments of various aspects of the mobile terminal 1200. For instance, the sensor component 1214 may detect an open/closed status of the mobile terminal 1200 and relative positioning of components (e.g. the display and the keypad of the mobile terminal 1200. The sensor component 514 may also detect a change in position of the mobile terminal 1200 or of a component in the mobile terminal 1200, a presence or absence of user contact with the mobile terminal 1200, an orientation or an acceleration/deceleration of the mobile terminal 1200, and a change in temperature of the mobile terminal 1200. The sensor component 1214 may include a proximity sensor configured to detect the presence of nearby objects without any physical contact. The sensor component 1214 may also include a illumination sensor, such as a CMOS or CCD image sensor, for use in imaging applications. In some embodiments, the sensor component 1214 may also include an accelerometer sensor, a gyroscope sensor, a magnetic sensor, a pressure sensor, or a temperature sensor.

The communication component 1216 is configured to facilitate wired or wireless communication between the mobile terminal 1200 and other devices. The mobile terminal 1200 can access a wireless network based on a communication standard, such as WIFI, 2G, or 3G, or a combination thereof. In one exemplary embodiment, the communication component 1216 receives a broadcast signal or broadcast associated information from an external broadcast management system via a broadcast channel. In one exemplary embodiment, the communication component 1216 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the mobile terminal 1200 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods.

In exemplary embodiments, there is also provided a non-transitory computer readable storage medium including instructions, such as the memory 1204 including instructions. The instructions may be performed by the processor 1220 of the mobile terminal 1200 so as to realize the method for generating information. For example, the non-transitory computer-readable storage medium may be a ROM, a RAM, a CD-ROM, a magnetic tape, a floppy disc, an optical data storage device, and the like.

Other embodiments of the disclosure will be apparent to those skilled in the art from consideration of the specification and practice of the disclosure disclosed here. This application is intended to cover any variations, uses, or adaptations of the disclosure following the general principles thereof and including such departures from the present disclosure as come within known or customary practice in the art.

## Claims

1. A plant monitor (140), comprising a main control chip (141; 241), a wireless communication chip (142; 242) connected with the main control chip (141; 241), and at least one growth environment monitoring sensor (143) connected with the main control chip (141; 241), wherein
the at least one growth environment monitoring sensor (143) comprises at least one of an illumination sensor (143a; 243), a temperature sensor (143b; 244), a humidity sensor (143c; 245), and a soil conductivity sensor (143d; 246);
the main control chip (141; 241) is configured to acquire growth environment data collected by the at least one growth environment monitoring sensor (143), and to send the growth environment data to a mobile terminal (120) via the wireless communication chip (142; 242), such that the mobile terminal (120) generates plant cultivation reference information according to the growth environment data.

2. The plant monitor (140) according to claim 1, further comprising:
an insertion arm (247) configured to be inserted into soil,
wherein the at least one growth environment monitoring sensor comprises a humidity sensor, and the humidity sensor (143c; 245) is arranged on the insertion arm (247), and configured to collect soil humidity.

3. The plant monitor (140) according to claim 1, further comprising:
an insertion arm (247) configured to be inserted into soil,
wherein the at least one growth environment monitoring sensor comprises a soil conductivity sensor, and the soil conductivity sensor (143c; 245) comprises two metal electrodes (246a), and the two metal electrodes (246a) are arranged on the insertion arm (247).

4. The plant monitor (140) according to claim 1, further comprising:
two insertion arms (247) configured to be inserted into soil,
wherein the soil conductivity sensor (143c; 245) comprises two metal electrodes (246a), the two insertion arms (247) being arranged in parallel, and each insertion arm (247) is provided with one metal electrode (246a).

5. The plant monitor (140) according to any one of claims 1 to 4, wherein
the main control chip (141; 241) is further configured to build a binding relationship with the mobile terminal (120) via the wireless communication chip (142; 242).

6. A method for generating information, comprising:
receiving growth environment data sent by a plant monitor (303; 405), wherein the growth environment data is collected by the plant monitor via a growth environment monitoring sensor, and the growth environment monitoring sensor comprises at least one of an illumination sensor, a temperature sensor, a humidity sensor, and a soil conductivity sensor; and
generating plant cultivation reference information according to the growth environment data (304).

7. The method according to claim 6, wherein generating plant cultivation reference information according to the growth environment data (304) comprises:
acquiring reference environment data corresponding to a predetermined plant (406);
comparing the growth environment data with the reference environment data (407); and
generating the plant cultivation reference information according to a comparison result (408).

8. The method according to claim 7, wherein the growth environment data comprises illumination data, and the reference environment data comprises a first threshold and a second threshold, and the first threshold is less than the second threshold; and
generating the plant cultivation reference information according to a comparison result (408) comprises:
when the illumination data or a statistic value of the illumination data is lower than the first threshold, generating first plant cultivation reference information, wherein the first plant cultivation reference information is configured to remind enhancing illumination (608a); and
when the illumination data or the statistic value of the illumination data is higher than the second threshold, generating second plant cultivation reference information, wherein the second plant cultivation reference information is configured to remind reducing illumination (608c).

9. The method according to claim 7, wherein the growth environment data comprises temperature data, and the reference environment data comprises a first temperature and a second temperature, and the first temperature is less than the second temperature; and
generating the plant cultivation reference information according to a comparison result (408) comprises:
when the temperature data or a statistic value of the temperature data is lower than the first temperature, generating third plant cultivation reference information, wherein the third plant cultivation reference information is configured to remind enhancing temperature (708a); and
when the temperature data or the statistic value of the temperature data is higher than the second temperature, generating fourth plant cultivation reference information, wherein the fourth plant cultivation reference information is configured to remind reducing temperature (708c).

10. The method according to claim 7, wherein the growth environment data comprises soil humidity data, and the reference environment data comprises a first humidity and a second humidity, and the first humidity is less than the second humidity; and
generating the plant cultivation reference information according to a comparison result (408) comprises:
when the soil humidity data or a statistic value of the soil humidity data is lower than the first humidity, generating fifth plant cultivation reference information, wherein the fifth plant cultivation reference information is configured to remind watering (808a); and
when the soil humidity data or the statistic value of the soil humidity data is higher than the second humidity, generating sixth plant cultivation reference information, wherein the sixth plant cultivation reference information is configured to remind ventilation (808c).

11. The method according to claim 7, wherein the growth environment data comprises a soil PH value, and the soil PH value is acquired by calculating according to the soil conductivity, and the reference environment data comprises a first PH value and a second PH value, and the first PH value is less than the second PH value; and
generating the plant cultivation reference information according to a comparison result (408) comprises:
when the soil PH value or a statistic value of the soil PH values is lower than the first PH value, generating seventh plant cultivation reference information, wherein the seventh plant cultivation reference information is configured to remind applying alkaline fertilizer (908a); and
when the soil PH value or the statistic value of the soil PH values is higher than the second PH value, generating eighth plant cultivation reference information, wherein the eighth plant cultivation reference information is configured to remind applying acidic fertilizer (908c).

12. The method according to claim 7, wherein the growth environment data comprises a soil PH value, and the soil PH value is acquired by calculating according to the soil conductivity, and the reference environment data comprises a third PH value and a fourth PH value, and the third PH value is less than the fourth PH value; and
generating the plant cultivation reference information according to a comparison result (408) comprises:
when the soil PH value or a statistic value of the soil PH values is lower than the third PH value, generating ninth plant cultivation reference information, wherein the ninth plant cultivation reference information is configured to remind applying first elemental fertilizer, and the first elemental fertilizer has an absorption rate lower than a predetermined condition in acidic soil (1008a); and
when the soil PH value or the statistic value of the soil PH values is higher than the fourth PH value, generating tenth plant cultivation reference information, wherein the tenth plant cultivation reference information is configured to remind applying second elemental fertilizer, and the second elemental fertilizer has an absorption rate lower than a predetermined condition in alkaline soil (1008c).

13. The method according to any one of claims 7 to 12, further comprising:
building a binding relationship with the plant monitor (401); and
recording the predetermined plant corresponding to the plant monitor (402).

14. The method according to any one of claims 6 to 12, further comprising:
displaying the plant cultivation reference information and a guidance button on a graphical user interface (409), wherein the guidance button comprises at least one of a button for buying a cultivation implement, a button for buying fertilizer, a button for jumping to a cultivation forum, and a button for jumping to a cultivation technique.

15. A computer program, which when executed on a processor of a mobile terminal, performs a method according to any one of claims 6-14.
